# EUROPEAN PATENT SPECIFICATION

(11) **EP 3 010 445 B1**
(45) Date of publication and mention of the grant of the patent: **07.06.2023**
(21) Application number: 14741446.0
(22) Date of filing: 16.06.2014
(51) Int. Cl.: A61F 2/16

(54) **SCLERAL FIXATION BAG**
SKLERALER FIXIERBEUTEL
POCHE DE FIXATION SCLÉRALE

(30) Priority: 16.06.2013 US 201313918970
(43) Date of publication of application: 27.04.2016
(73) Proprietor: VisionCare, Inc., Saratoga, CA (US)
(72) Inventor: AHARONI, Eli, 6940005 Tel Aviv (IL)
(74) Representative: Jaeger, Michael David
(86) International application number: PCT/US2014/042458
(87) International publication number: WO 2014/204827

(56) References cited:
- US-A- 4 409 690
- US-A- 5 628 795
- US-A- 6 152 959
- US-A1- 2008 004 699
- US-A1- 2013 116 781

## Description

### FIELD OF THE INVENTION

The present invention relates generally to intraocular devices, and particularly to a scleral fixation bag, which can be used, inter alia, to mount therein intraocular lenses (IOLs) when no intact capsular bag is present.

### BACKGROUND OF THE INVENTION

In a typical cataract surgery, the natural lens is removed by phacoemulsification from the eye. The lens material is broken up and vacuumed out of the eye, but the capsular bag is left generally intact. The remaining capsular bag is very important because it houses and supports an IOL, which can be acted on by the zonules and ciliary muscles for possible accommodation.

However, the capsular bag does not always provide sufficient support for the IOL. When performing cataract surgery for eyes with severe zonular dehiscence or lens subluxation, surgeons need to employ a surgical technique to fixate an intraocular lens (IOL) without adequate capsular support. Several methods have been employed by surgeons to address the problem of insufficient capsular support. One method is implantation of an open-loop anterior chamber IOL. Another method involves iris suture fixation or scleral suture fixation of a posterior chamber IOL. Yet another method involves scleral fixation of a capsular tension ring with the IOL.

However, all of these methods are applicable when the capsular bag is still intact. There are situations when no capsular bag is present. For example, a lens exchange is required to replace an IOL with another IOL. Removing the first IOL can severely damage the capsular bag to the point where a remnant bag or no bag is present for holding the replacement IOL.

US patent publication No. 2008/0004699 relates to an accommodating intraocular lens (AIOL) assembly including a haptics system for self-anchoring implantation in a human eye's annular ciliary sulcus for retaining an AIOL at a desired position along the human eye's visual axis.

US patent publication No. 6152959 relates to an iris fixated intraocular lens for implanting in the anterior chamber of an eye which includes an optic having an optical axis and anterior and posterior sides, and first and second fixation members, each of the fixation members having a proximal end region and a distal end region.

US-A-2013/116781 describes an accommodating intraocular lens (AIOL) assembly including a discrete pre-assembled monolithic AIOL assemblage and a discrete haptics system having a haptics ring and at least two elongated C-shaped haptics for self-anchoring in a human scleral wall at the ciliary sulcus. The AIOL assemblages include an AIOL capsule and an integrally formed base member. The AIOL assemblages also include an annular haptics support surround posterior to an anterior structure on implantation in a human eye of a supine human. AIOL assemblies are assembled in situ by mounting a haptics system onto a previously implanted AIOL assemblage.

### SUMMARY OF THE INVENTION

The present invention seeks to provide improved devices for mounting IOLs or other intraocular devices even if no capsular bag is present. As is described further in detail hereinbelow, the invention provides a scleral fixation bag which serves as a mounting platform for intraocular devices, even if no natural bag is present.

The present invention relates to a device as set forth in the appended claims. In particular it relates to an intraocular device comprising: a scleral fixation bag comprising haptics that extend therefrom, each of said haptics comprising a slender loop element with a proximal end attached to said scleral fixation bag, said slender loop element having a length, width and thickness, the length following a curved path and the width being generally parallel to a central anterior-posterior axis of said scleral fixation bag characterised by a distal hook portion extending from a distal end of said slender loop element, said distal hook portion comprising a surface that extends between an anterior edge and a posterior edge, said distal hook portion being tilted with respect to the width and to the thickness of said slender loop element towards the central anterior-posterior axis of said scleral fixation bag so that said anterior edge is radially more proximal than said posterior edge with respect to said central anterior-posterior axis.

The distal hook portion is round at its distal tip and tapers to be thinner from where it extends from the slender loop element.

The distal hook portion is thinner than the slender loop element.

The thickness of the slender loop elements is less than the width.

The distal hook portion is formed with a through hole.

The proximal end of the slender loop element is generally tangential to an outer periphery of the scleral fixation bag.

The distal hook portion and the slender loop element have different rigidity.

The slender loop element and the scleral fixation bag have different rigidity.

The anterior-most surface of the distal hook portion is not posterior to an anterior-most surface of the intraocular device.

The anterior-most surface of the slender loop element is not posterior to an anterior-most surface of the scleral fixation bag.

The scleral fixation bag includes a channel for receiving therein an IOL.

### BRIEF DESCRIPTION OF THE DRAWINGS

The present invention will be understood and appreciated more fully from the following detailed description taken in conjunction with the appended drawings in which:
Fig. 1 is a simplified pictorial illustration of a scleral fixation bag with haptics, constructed and operative in accordance with an embodiment of the present invention;
Fig. 2 is a simplified illustration of the scleral fixation bag of Fig. 1 implanted in an eye, viewed transverse to the anterior-posterior axis, wherein the haptics are fixed in a curved sclerotomy, in accordance with an embodiment of the present invention;
Fig. 3 is a simplified side-view illustration of a portion of the scleral fixation bag;
Fig. 4 is a simplified side-view illustration of an intraocular device (IMT) mounted in the scleral fixation bag, in accordance with an embodiment of the present invention;
Fig. 5 is a simplified pictorial illustration of a scleral fixation bag with haptics;
Fig. 6 is a simplified pictorial illustration of an IOL mounted in the scleral fixation bag that cooperates with another IOL mounted in the posterior chamber, e.g., in the ciliary muscles; and
Fig. 6A is an enlarged view of a portion of the fixation bag, showing a posterior platform attached to the scleral fixation bag.

### DETAILED DESCRIPTION OF THE PRESENT INVENTION

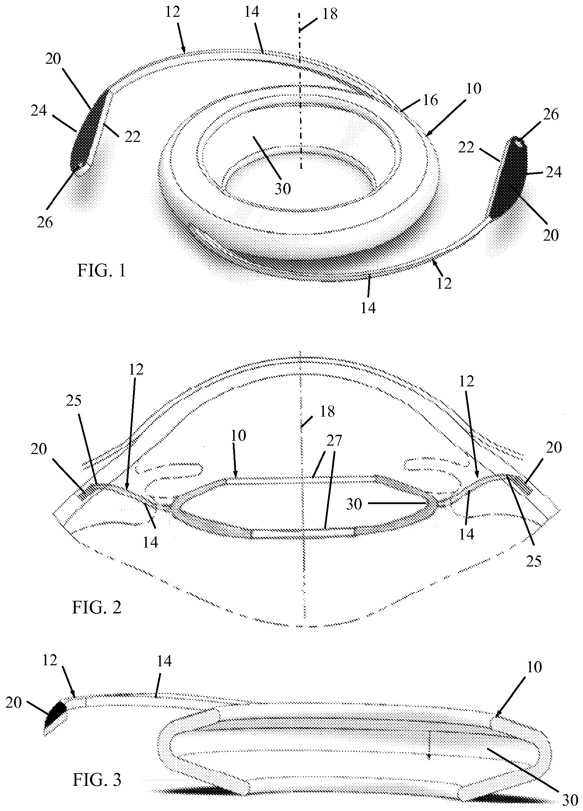

Reference is now made to Figs. 1-3, which illustrate a scleral fixation bag 10 with haptics 12, constructed and operative in accordance with an embodiment of the present invention. Scleral fixation bag 10 is shown as a ring, but it may have other shapes as well.

Each haptic 12 includes a slender loop element 14 with a proximal end 16 attached to scleral fixation bag 10. Slender loop element 14 has a length, width and thickness; its length follows a curved path and its width is generally parallel to a central anterior-posterior axis 18 of scleral fixation bag 10. The outer diameter of scleral fixation bag 10 with haptics 12 is, without limitation, 15 mm for proper mounting in the sclera.

A distal hook portion 20 extends from a distal end of slender loop element 14. Distal hook portion 20 includes an anterior edge 22 tilted towards the central anterior-posterior axis 18 of scleral fixation bag 10. Preferably, but not necessarily, distal hook portion 24 is flat. The shape of distal hook portion 24 may match the shape of its insertion place in the sclera. Distal hook portion 24 is rounded at its distal tip and tapers to be thinner from where it extends from slender loop element 18. Distal hook portion 24 may be formed with a through hole 26, for grasping or dialing or other adjustments. Through hole 26 is preferably central and not shifted to one side. In one embodiment, distal hook portion 20 is thinner than slender loop element 14. In one embodiment, the thickness of slender loop elements 14 is less than its width; in other embodiments they are equal. The distal hook portion may be connected to a suture (not shown) in different surgical procedures.

The proximal end of slender loop element 14 is generally tangential to the outer periphery of scleral fixation bag 10.

The scleral fixation bag 10 and haptics 12 may be made from the same or different materials, and each may be made of one or more materials. The materials used are biocompatible and optically transparent, and can be hydrophilic or hydrophobic. The material may be rigid or flexible, hard or soft, such as without limitation, methacrylates (e.g., polymethyl methacrylate), olefins (e.g., polypropylene), and silicones. For example, without limitation, scleral fixation bag 10 may be made of silicone whereas haptics 12 may be made of PMMA.

The distal hook portion 20 and the slender loop element 14 can have different rigidity. Similarly, slender loop element 14 and scleral fixation bag 10 can have different rigidity. Fixation bag 10 can be made of a flexible folding material for easy insertion during the surgical procedure.

It is noted that the anterior-most surface of distal hook portion 20 is not posterior to the anterior-most surface of scleral fixation bag 10 (that is, it is either flush with it or more anterior). Similarly, the anterior-most surface of the slender loop element 14 is not posterior to the anterior-most surface of scleral fixation bag 10.

Scleral fixation bag 10 includes an open central aperture 27 (Fig. 2).

In accordance with an embodiment of the present invention the scleral fixation bag 10 includes a channel 30 for receiving therein an intraocular device, such as an intraocular lens (IOL) or implantable telescope, not shown. Channel 30 may extend completely around the inner periphery of scleral fixation bag 10 or may extend over one or more discrete angular segments of the inner periphery of scleral fixation bag 10.

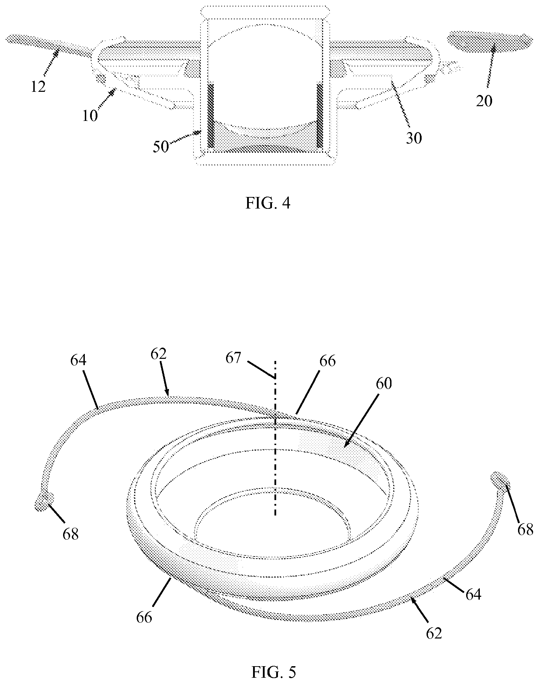

The scleral fixation bag 10 can be introduced through an incision in the limbus, for example. The haptics 12 are fixed in curved sclerotomies 25 and properly center the fixation bag 10. An IOL (such as, but not limited to, a monofocal IOL, multifocal IOL, accommodating IOL and others) or other intraocular device (such as an implantable miniature telescope (IMT) 50, seen in Fig. 4) can be easily mounted in channel 30. The fixation bag 10 is held properly by the haptics 12 in sclerotomies 25, even without any capsular bag being present.

Reference is now made to Fig. 5, which illustrates a scleral fixation bag 60 with haptics 62, constructed and operative in accordance with another arrangement. As in the embodiments of the invention, each haptic 62 includes a slender loop element 64 with a proximal end 66 attached to scleral fixation bag 60 and a distal hook portion 68 whose anterior edge is tilted towards the central anterior-posterior axis 67 of scleral fixation bag 60. In this example, slender loop element 64 is a slender wire and distal hook portion 68 is a rounded eyelet bent or otherwise formed from the end of the slender wire.

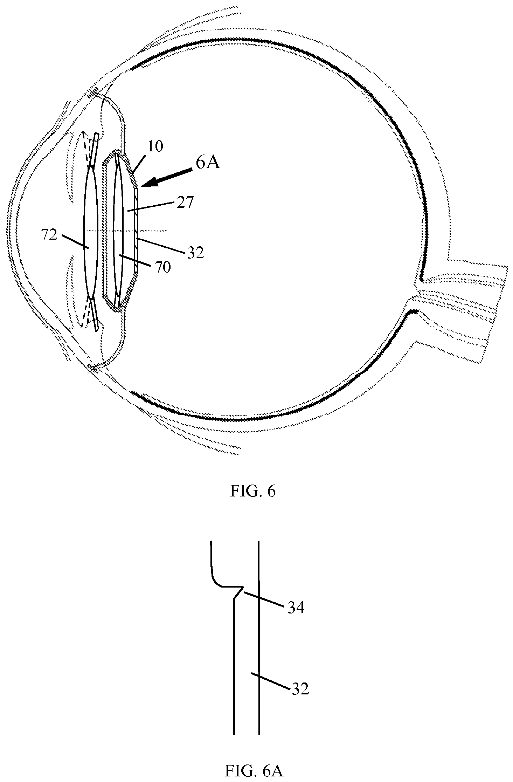

The scleral fixation bag of the present invention can be useful in providing a relatively static platform for mounting therein an IOL, which can be used in conjunction with another IOL mounted posterior to the iris and anterior to the scleral fixation bag, such as in the ciliary muscles, ciliary process or ciliary sulcus. This creates a doublet lens with superior accommodative power than previous lenses. This is because the posterior of the two lenses, that is, the lens mounted in the scleral fixation bag, is held much more statically than the anterior of the two lenses; thus the anterior lens moves much more with respect to the posterior lens than prior art doublet IOLs which are both held in the capsular bag and have much less relative movement because both lenses move. Such an embodiment is illustrated in Fig. 6, wherein an IOL 70 is mounted in scleral fixation bag 10 and another IOL 72 is mounted in the posterior chamber posterior to the iris and anterior to the scleral fixation bag 10, such as in the ciliary muscles, or even in the ciliary sulcus (indicated by broken lines).

Fig. 6 also illustrates another option, namely a posterior platform 32 attached to the scleral fixation bag 10. Platform 32 may be used to close the open central aperture 27 of bag 10. This may help prevent migration of vitreous humor through the fixation bag 10. Platform 32 may be formed with an annular groove 34 (seen in the enlarged view of Fig. 6A), so that the portion of platform 32 which covers aperture 27 may be broken off, if desired for a particular application. Platform 32 may have optical power (positive or negative). Platform 32 and scleral fixation bag 10 may be made from the same or different materials. Platform 32 may also be useful in the doublet lens configuration of Fig. 6, in which the platform helps keep the lens static in the fixation bag.

## Claims

1. An intraocular device comprising:
a scleral fixation bag (10) comprising haptics (12) that extend therefrom, each of said haptics (12) comprising a slender loop element (14) with a proximal end attached to said scleral fixation bag (10), said slender loop element (14) having a length, width and thickness, the length following a curved path and the width being generally parallel to a central anterior-posterior axis (18) of said scleral fixation bag (10),
**characterised by** a distal hook portion (20) extending from a distal end of said slender loop element (14), said distal hook portion (20) comprising a surface that extends between an anterior edge (22) and a posterior edge (24), said distal hook portion (20) being tilted with respect to the width and to the thickness of said slender loop element (14) towards the central anterior-posterior axis (18) of said scleral fixation bag (10) so that said anterior edge (22) is radially more proximal than said posterior edge (24) with respect to said central anterior-posterior axis (18).

2. The intraocular device according to claim 1, wherein said distal hook portion (20) is rounded at its distal tip and tapers to be thinner from where it extends from said slender loop element (14).

3. The intraocular device according to claim 1, wherein the thickness of said slender loop element (14) is less than the width.

4. The intraocular device according to claim 1, wherein said distal hook portion (20) is formed with a central through hole (26).

5. The intraocular device according to claim 1, wherein said distal hook portion (20) and said slender loop element (14) have different rigidity.

6. The intraocular device according to claim 1, wherein an anterior-most surface of said distal hook portion (20) is not posterior to an anterior-most surface of said intraocular device.

7. The intraocular device according to claim 1, wherein said scleral fixation bag (10) comprises a channel (30) for receiving therein a different intraocular device.

8. The intraocular device according to claim 1, wherein a posterior platform (32) is attached to said scleral fixation bag (10).

9. The intraocular device according to claim 8, wherein said posterior platform (32) is formed with an annular groove (34).

10. The intraocular device according to claim 8, wherein said posterior platform (32) has optical power.

## Patentansprüche

1. Intraokulare Vorrichtung, umfassend:
einen skleralen Fixierbeutel (10), der Haptiken (12) umfasst, die sich davon erstrecken, wobei jede der Haptiken (12) ein schmales Schleifenelement (14) mit einem nahen Ende umfasst, das am skleralen Fixierbeutel (10) angebracht ist, wobei das schmale Schleifenelement (14) eine Länge, Breite und Dicke aufweist, wobei die Länge einem gekrümmten Weg folgt und die Breite im Allgemeinen parallel zu einer zentralen Vorne-Hinten-Achse (18) des skleralen Fixierbeutels (10) verläuft,
**gekennzeichnet durch** einen fernen Hakenabschnitt (20), der sich von einem fernen Ende des schmalen Schleifenelements (14) erstreckt, wobei der ferne Hakenabschnitt (20) eine Fläche umfasst, die sich zwischen einer Vorderkante (22) und einer Hinterkante (24) erstreckt, wobei der ferne Hakenabschnitt (20) in Bezug auf die Breite und die Dicke des schmalen Schleifenelements (14) hin zur zentralen Vorne-Hinten-Achse (18) des skleralen Fixierbeutels (10) derart geneigt ist, dass sich die Vorderkante (22) radial näher als die Hinterkante (24) in Bezug auf die Vorne-Hinten-Achse (18) befindet.

2. Intraokulare Vorrichtung nach Anspruch 1, wobei der ferne Hakenabschnitt (20) an seiner fernen Spitze abgerundet ist und sich so verjüngt, dass er dünner ist von dort, wo er sich von dem schmalen Schleifenelement (14) erstreckt.

3. Intraokulare Vorrichtung nach Anspruch 1, wobei die Dicke des schmalen Schleifenelements (14) geringer als die Breite ist.

4. Intraokulare Vorrichtung nach Anspruch 1, wobei der ferne Hakenabschnitt (20) mit einem zentralen Durchgangsloch (26) ausgebildet ist.

5. Intraokulare Vorrichtung nach Anspruch 1, wobei der ferne Hakenabschnitt (20) und das schmale Schleifenelement (14) unterschiedliche Steifigkeit aufweisen.

6. Intraokulare Vorrichtung nach Anspruch 1, wobei eine vorderste Fläche des distalen Hakenabschnitts (20) nicht hinter einer vordersten Fläche der intraokularen Vorrichtung liegt.

7. Intraokulare Vorrichtung nach Anspruch 1, wobei der sklerale Fixierbeutel (10) einen Kanal (30) zum Aufnehmen einer anderen intraokularen Vorrichtung darin umfasst.

8. Intraokulare Vorrichtung nach Anspruch 1, wobei an dem skleralen Fixierbeutel (10) eine hintere Plattform (32) angebracht ist.

9. Intraokulare Vorrichtung nach Anspruch 8, wobei die hintere Plattform (32) mit einer ringförmigen Nut (34) ausgebildet ist.

10. Intraokulare Vorrichtung nach Anspruch 8, wobei die hintere Plattform (32) eine optische Leistung aufweist.

## Revendications

1. Un dispositif intraoculaire comprenant :
une poche de fixation sclérale (10) comprenant des haptiques (12) s'étendant à partir de cette dernière, chacun des haptiques (12) comprenant un élément en boucle mince (14) avec une extrémité proximale fixée à ladite poche de fixation sclérale (10), ledit élément en boucle mince (14) ayant un longueur, une largeur et un épaisseur, la longueur suivant une trajectoire incurvée et la largeur étant généralement parallèle à un axe central antéro-postérieur (18) de ladite poche de fixation sclérale (10),
**caractérisé par** une partie de crochet distale (20) s'étendant à partir d'une extrémité distale dudit élément en boucle mince (14), ladite partie de crochet distale (20) comprenant une surface qui s'étend entre un bord antérieur (22) et un bord postérieur (24), la partie de crochet distale (20) étant inclinée par rapport à la largeur et à l'épaisseur dudit élément en boucle mince (14) vers l'axe central antéropostérieur (18) de ladite poche de fixation sclérale (10) de sorte que ledit bord antérieur (22) soit radialement plus proximal que ledit bord postérieur (24) par rapport audit axe central antéropostérieur (18).

2. Dispositif intraoculaire selon la revendication 1, dans lequel ladite partie de crochet distale (20) est arrondie au niveau de sa pointe distale et se rétrécit pour être plus mince à partir de l'endroit où elle s'étend à partir dudit élément de boucle mince (14).

3. Dispositif intraoculaire selon la revendication 1, dans lequel l'épaisseur dudit élément de boucle mince (14) est inférieure à la largeur.

4. Dispositif intraoculaire selon la revendication 1, dans lequel ladite partie de crochet distale (20) est formée avec un trou traversant central (26).

5. Dispositif intraoculaire selon la revendication 1, dans lequel ladite partie de crochet distale (20) et ledit élément de boucle mince (14) ont une rigidité différente.

6. Dispositif intraoculaire selon la revendication 1, dans lequel une surface la plus antérieure de ladite partie de crochet distale (20) n'est pas postérieure à une surface la plus antérieure dudit dispositif intraoculaire.

7. Dispositif intraoculaire selon la revendication 1, dans lequel ladite poche de fixation sclérale (10)) comprend un canal (30) pour recevoir en son sein un dispositif intraoculaire différent.

8. Dispositif intraoculaire selon la revendication 1, dans lequel une plateforme postérieure (32) est fixée à ladite poche de fixation sclérale (10).

9. Dispositif intraoculaire selon la revendication 8, dans lequel ladite plate-forme postérieure (32) est formée avec une rainure annulaire (34).

10. Dispositif intraoculaire selon la revendication 8, dans lequel ladite plate-forme postérieure (32) a une puissance optique.
